# EUROPEAN PATENT SPECIFICATION

(11) **EP 1 982 694 B1**
(45) Date of publication and mention of the grant of the patent: **19.08.2015**
(21) Application number: 07425234.7
(22) Date of filing: 20.04.2007
(51) Int. Cl.: A61K 8/00, A61K 47/06, A61K 31/06, A61K 9/06, A61K 47/44

(54) **Anti-oedema composition**
Antiödemzusammensetzung
Composition anti-'dème

(43) Date of publication of application: 22.10.2008
(73) Proprietor: D.M.G. Italia Srl, 00040 Pomezia (RM) (IT)
(72) Inventor: Mercuri, Luigi, Pomezia 00044 (IT)
(74) Representative: Sarpi, Maurizio

(56) References cited:
- WO-A-2005/009448
- DE-A1- 19 718 848
- GB-A- 2 321 405
- DATABASE CA [Online] CHEMICAL ABSTRACTS SERVICE, COLUMBUS, OHIO, US; CHEN, JUN: "Adsorbed burn ointment" XP002463640 retrieved from STN Database accession no. 136:330542 & CN 1 306 825 A (PEOP. REP. CHINA) 8 August 2001 (2001-08-08)

## Description

The present invention refers to the pharmaceutical field and more particularly to a composition for the physic-osmotic treatment of oedematous states, also of traumatic origin, comprising an osmotic agent and pharmaceutically acceptable vehicles, excipient and preservatives.

As it is well known the oedema is a swelling due to the accumulation of liquids in the interstitial spaces.

Nowadays, the oedemas of soft tissues are topically treated by local application of drugs containing glucocorticoids or enzymes which reduce inflammation ad help the oedema absorption.

Unfortunately, said compounds have many side effects. For instance, the prolonged or excessive use of glucocorticoids may influence bone metabolism or may cause muscle atrophy or may promote acne, infections, atrophic streaking and dermatitis.

On the other hand, the use of enzymes, which degrade the fibrous components of oedema and help clot dissolution, is not indicated in the presence of concomitant infections.

In both cases, the, topical or not-topical, use of said drugs is unadvisable in children, during pregnancy and breast-feeding.

The aim of the present invention is to provide an alternative composition which can effectively help the reduction of oedemas, likewise traumatic oedemas, also in combination with sprains, bruises, muscle strains etc. without showing the typical side effects, that can appear in subjects being intolerant to the active agent, or becoming intolerant to the active agent after its prolonged use.

Therefore, the object of the present invention is a composition comprising an osmotic agent as the active agent, which preferably is selected form the group consisting of sodium chloride, , magnesium chloride, potassium chloride and sea salt, and pharmaceutically acceptable vehicles, excipient and preservatives.

The use of said salts in topical compositions is already known in the art.
Patent N. US 6.706.279 discloses a composition comprising a hydrogel matrix wherein a colloidal moistening agent, a salt and a polysaccharide are dissolved. The salt, which can be NaCl, MgCl₂ or sea salt exert a disinfecting action. Said product is used for promoting wound healing.

The International application N. WO 2005/009448 describes a system for the treatment of suppurative and inflammated wounds wherein in a matrix in the form of micro-granules, a salt, an antiseptic agent and an antioxidant agent are embedded.
the composition of the present invention is not disclosed in the cited documents.

Moreover, the two cited documents refer to the treatment of wounds wherein the presence of lacerations and/or abrasions causes bleeding, contamination of bacterium and dirty, and related infections.

GB2321405 refers to the cosmetic field and discloses a body polisher which contains sea salt plus an emollient and skin conditioner, in particular, that in the form of silicone oils such as dimethicone and fragrance.

Also DE19718848 refers to the cosmetic field and discloses an anti-cellulite ointment comprising orange oil, sea salt and whey powder.

CN1306825 discloses an ointment composed of 10-20% of activated carbon and 80-90% hydrophilic substrate, such as Vaseline-lanolin, wherein the activated carbon contains 0.5-10% of osmotic and isotonic regulator, such as NaCl, which is used in repairing burnt wounds.

It can be easily derived from the cited documents belonging to the state of the art that wounds need an anti-inflammatory, an antibacterial and a painrelief treatment at the same time.

On the contrary, the composition of the present invention has been formulated with the intend of treating oedemas that show an accumulation of liquids in the interstitial spaces without concomitant laceration or exudation, thus, said use does not belong to the state of the art.

Therefore is an object of the present invention a composition comprising an osmotic agent as the active agent and pharmaceutically acceptable vehicles, excipients and preservatives.

The active agent is in a concentration from 100 g to 800/ Kg of product, preferably in a concentration between 200 g and 700 g/Kg of product, and more preferably from 400 g to 600 g /Kg of product.

The composition object of the present invention, being able to reduce traumatic or non-traumatic oedema thanks to the physic-osmotic properties of the active agents thereof, solves the problem of reducing the side effects of other medicaments available on the market.

In fact, it has been demonstrated that, in the composition object of the present invention, the high concentration of the osmotic agent drains the liquids that accumulate in the interstitial spaces in the form of a swelling, and helps the oedema reduction, without acting in a pharmaceutical manner and without side effects.

In a preferred embodiment the active agent is at least a salt selected from the group consisting of NaCl, MgCl₂, KCl and sea salt.

The composition object of the present invention is in a pharmaceutical form for dermatologic use, as an ointment, cream, emulsion, gel or paste to be applied on the skin to obtain the above action.

The person skilled in the art will be able to choose suitable oily or hydrophilic excipients and emulsifiers to be added to the active agent for the preparation of the above mentioned pharmaceutical forms.

In a preferred embodiment, the composition of the present invention is in the form of an ointment by adding suitable oily excipients as triglicerides mixtures (glycerine esters with C₁₂-C₁₈ fatty acids), waxes (fatty acids esters with superior aliphatic alcohol), hydrocarbons (vaseline, vaseline oil, paraffin), silicones and mixtures thereof, or hydrophilic excipients as lanolin or mixtures of oily and hydrophilic excipients, according to the common pharmaceutical technique.

In a particularly preferred embodiment, the composition in the form of an ointment comprises sodium chloride as the active agent, white vaseline and vaseline oil as excipients and further comprises chlorobutanol, BHT, enotera oil and mint.

According to the common industrial practice, the ointment will be prepared by mechanical incorporation, by refining or by casting.

In the first case, the active agent will be introduced and distributed in the excipient by mechanical stirring. The process can be preceded by a step wherein the incorporation of the insoluble ingredient in the excipient is helped by incorporating a little portion of the same excipient and diluting the same in subsequent concentrations. A more homogeneous preparation may be obtained by a further refining step.

In the preparation by casting the components of the excipient are fused together and then cooled under stirring; the active agent will be then suspended in the fused excipient, and gelify under stirring.

### Example 1

In an embodiment, the composition of the present invention is in the form of an ointment having the following quantitative formulation for 1 kg of product:

| | |
|---|---|
| White Vaseline | 326.6 g |
| Vaseline oil | 115 g |
| Sodium chloride | 500 g |
| Enotera oil | 50 g |
| Mint o.e. | 0.2 g |
| BHT | 0.2 g |
| Chlorobutanol | 8 g |

## Claims

1. Ointment comprising:
White vaseline
Vaseline oil
Sodium chloride
Enotera oil
Mint
BHT Chlorobutanol

2. The ointment of claim 1 comprising for kg of product:
| | |
|---|---|
| White vaseline | 326,6 g |
| Vaseline oil | 115 g |
| Sodium chloride | 500 g |
| Enotera oil | 50 g |
| Mint | 0.2 g |
| BHT | 0.2 g |
| Chlorobutanol | 8 g |

## Patentansprüche

1. Salbe, umfassend:
Weißes Vaselin
Vaselinöl
Natriumchlorid
Nachtkerzenöl
Minze
BHT
Chlorbutanol

2. Die Salbe aus Anspruch 1, umfassend pro kg des Produktes:
| | |
|---|---|
| Weißes Vaselin | 326,6 g |
| Vaselinöl | 115 g |
| Natriumchlorid | 500 g |
| Nachtkerzenöl | 50 g |
| Minze | 0,2 g |
| BHT | 0,2 g |
| Chlorbutanol | 8 g |

## Revendications

1. Pommade comprenant :
Vaseline blanche
Huile de vaseline
Chlorure de sodium
Huile d'onagre
Menthe
BHT
Chlorobutanol

2. Pommade selon la revendication 1, comprenant par kg de produis :
| | |
|---|---|
| Vaseline blanche | 326,6 g |
| Huile de vaseline | 115 g |
| Chlorure de sodium | 500 g |
| Huile d'onagre | 50 g |
| Menthe | 0,2 g |
| BHT | 0,2 g |
| Chlorobutanol | 8 g |
